# EUROPEAN PATENT SPECIFICATION

(11) **EP 3 868 443 B1**
(45) Date of publication and mention of the grant of the patent: **15.11.2023**
(21) Application number: 20195631.5
(22) Date of filing: 11.09.2020
(51) Int. Cl.: A61K 36/03, A61P 27/02

(54) **COMPOSITION COMPRISING TISOCHRYSIS LUTEA FOR USE IN PREVENTING OR TREATING DRY EYE SYNDROME**
TISOCHRYSIS LUTEA-ENTHALTEND ZUSAMMENSETZUNG ZUR VORBEUGUNG ODER BEHANDLUNG VON TROCKENE-AUGEN-SYNDROM
COMPOSITION COMPRENANT TISOCHRYSIS LUTEA POUR LA PRÉVENTION OU LE TRAITEMENT DU SYNDROME DE L'OEIL SEC

(30) Priority: 21.02.2020 KR 20200021760
(43) Date of publication of application: 25.08.2021
(73) Proprietor: Korea Institute of Science and Technology, Seoul 02792 (KR)
(72) Inventor: PAN, Cheol-Ho, 25451 Gangwon-do (KR); KIM, Jin-Chul, 25451 Gangwon-do (KR); PARK, Keunwan, 25451 Gangwon-do (KR); LEE, Eun Ha, 25451 Gangwon-do (KR)
(74) Representative: advotec.

(56) References cited:
- EP-A1- 2 918 278
- MOHAMADNIA SONIA ET AL: "Production of fucoxanthin by the microalga Tisochrysis lutea: A review of recent developments", AQUACULTURE, vol. 516, 1 February 2020 (2020-02-01), page 734637, XP055777943, Amsterdam, NL ISSN: 0044-8486, DOI: 10.1016/j.aquaculture.2019.734637
- SANG MIN KIM ET AL: "Fucoxanthin as a major carotenoid in Isochrysis aff. galbana: Characterization of extraction for commercial application", JOURNAL OF THE KOREAN SOCIETY FOR APPLIED BIOLOGICAL CHEMISTRY, vol. 55, no. 4, 1 August 2012 (2012-08-01) , pages 477-483, XP055049996, ISSN: 1738-2203, DOI: 10.1007/s13765-012-2108-3
- CHEN SHIU-JAU ET AL: "Protective Effects of Fucoxanthin on Ultraviolet B-Induced Corneal Denervation and Inflammatory Pain in a Rat Model", MARINE DRUGS, vol. 17, no. 3, 5 March 2019 (2019-03-05), page 152, XP055777968, DOI: 10.3390/md17030152 Retrieved from the Internet: URL:https://www.ncbi.nlm.nih.gov/pmc/artic les/PMC6471339/pdf/marinedrugs-17-00152.pd f>
- YIXIANG LIU ET AL: "Protective Effect of Fucoxanthin Isolated from Laminaria japonica against Visible Light-Induced Retinal Damage Both in Vitro and in Vivo", JOURNAL OF AGRICULTURAL AND FOOD CHEMISTRY, vol. 64, no. 2, 4 January 2016 (2016-01-04), pages 416-424, XP055615464, US ISSN: 0021-8561, DOI: 10.1021/acs.jafc.5b05436

## Description

### CROSS-REFERENCE TO RELATED APPLICATION

This application claims the benefit of Korean Patent Application No. 10-2020-0021760, filed on February 21, 2020, in the Korean Intellectual Property Office.

### BACKGROUND

### 1. Field

The present disclosure relates to a pharmaceutical composition including *Tisochrysis lutea* as an active ingredient, for preventing or treating an eye disease.

### 2. Description of Related Art

As modern society is said to be a multimedia era and a visual era, modern society's dependence on vision is increasing, and use of eyes in smartphones, TVs, computers, electronic games, and workplaces is increasing. For examinees, excessive study and excessive use of computers have increased eye fatigue. In particular, the use of smartphones, development of computers, and long-term use of eyes by examinees are causes of eye strain, and the eyes are the primary organs in the body that may become easily fatigued. One of the most common causes of eye strain is stress. Stress, which is always mentioned in all common senses about health, makes eyes more fatigued. Most young people have such experiences. The number of people complaining of eye fatigue and headaches with dry eye syndrome is increasing exponentially, since eyes are being overworked due to the excessive use of computers and smartphones.

Dry eye syndrome is a disease that includes damage to the eyeball surface due to an improper balance of tear components resulting from a lack of tears or excessive evaporation of tears, and is accompanied by symptoms including eye irritation such as foreign body sensation and sensation of dryness, as well as a deterioration of eyesight. This dry eye syndrome affects quality of life by reducing visual function and making it difficult to perform everyday activities such as driving, reading, watching television, etc.

In most cases of dry eye syndrome, corneal/conjunctival disorders are caused by abnormalities in one of the oily layer, the aqueous layer, and the mucous layer, which make up the tear film. Among them, abnormality of the mucous layer causes serious corneal disorders. Dry eye syndrome increases fluorescein permeability of corneal epithelial cells, conjunctival transformation, and loss of goblet cells, leading to pathological changes in epithelial cells of the corneal surface. As a result, corneal epithelial disorders or corneal epithelial erosion as well as corneal ulceration and eye infections may occur, and in some cases, corneal transplantation is needed.

Even though the number of patients with dry eye syndrome continues to increase due to the use of electronic devices such as computers and smartphones, as well as increased use of contact lenses and increases in fine dust, there are no specific therapeutic agents or functional foods.

### PRIOR ART DOCUMENTS

### PATENT DOCUMENTS

(Patent Document 1) Korean Patent No. KR10-2040810

MOHAMADNIA SONIA ET AL: "Production of fucoxanthin by the microalga Tisochrysis lutea: A review of recent developments", AQUACULTURE, vol. 516, 1 February 2020 (2020-02-01), page 734637, and SANG MIN KIM ET AL: "Fucoxanthin as a major carotenoid in Isochrysis aff. galbana: Characterization of extraction for commercial application", JOURNAL OF THE KOREAN SOCIETY FOR APPLIED BIOLOGICAL CHEMISTRY, vol. 55, no. 4, 1 August 2012 (2012-08-01), pages 477-483, disclose beneficial effects of fucoxanthin as an anti-cancer, anti-oxidant and anti-angiogenic agent. Furthermore, these documents disclose that T. lutea has traditionally been used as feed for aquaculture.

EP 2 918 278 A1 discloses a use of microalgae-extracted fucoxanthin as a nutraceutical.

CHEN SHIU-JAU ET AL: "Protective Effects of Fucoxanthin on Ultraviolet B-Induced Corneal Denervation and Inflammatory Pain in a Rat Model", MARINE DRUGS, vol. 17, no. 3, 5 March 2019 (1029-03-05), page 152, and YIXIANG LIU ET AL: "Protective Effect of Fucoxanthin Isolated from Laminaria japonica against Visible Light-Induced Retinal Damage Both in Vitro and in Vivo", JOURNAL OF AGRICULTURAL AND FOOD CHEMISTRY, vol. 64, no. 2, 4 January 2016 (2016-01-04), pages 416-424, disclose that fucoxanthin protects the cornea from inflammation and is effective in a model of keratitis and retinal damage.

### SUMMARY

The invention provides a composition comprising *Tisochrysis lutea* for use in preventing or treating a dry eye syndrome.

Further, described is a method of treating an eye disease in an individual, the method including administering, to the individual, the pharmaceutical composition in an effective amount for preventing or treating the eye disease.

Further described is a health functional food composition for preventing or improving an eye disease or for improving eye function, the health functional food composition including *Tisochrysis lutea* as an active ingredient.

Additional aspects will be set forth in part in the description which follows and, in part, will be apparent from the description, or may be learned by practice of the presented embodiments of the disclosure.

An aspect provides a pharmaceutical composition for preventing or treating dry eye syndrome the pharmaceutical composition including *Tisochrysis lutea* as an active ingredient.

The *Tisochrysis lutea* is an algae that commonly inhabits in the aquatic environment. Further, the *Tisochrysis lutea* is a protist that has a photosynthetic pigment and has an autotrophic lifestyle.

The *Tisochrysis lutea* may be included in an amount of 0.0001% by weight to 99.0% by weight, for example, 0.01% by weight to 60% by weight, 0.01% by weight to 40% by weight, 0.01% by weight to 30% by weight, 0.01% by weight to 20% by weight, 0.01% by weight to 10% by weight, 0.01% by weight to 5% by weight, 0.05% by weight to 60% by weight, 0.05% by weight to 40% by weight, 0.05% by weight to 30% by weight, 0.05% by weight to 20% by weight, 0.05% by weight to 10% by weight, 0.05% by weight to 5% by weight, 0.1% by weight to 60% by weight, 0.1% by weight to 40% by weight, 0.1% by weight to 30% by weight, 0.1% by weight to 20% by weight, 0.1% by weight to 10% by weight, or 0.1% by weight to 5% by weight, based on the total weight of the composition.

The eye disease is dry eye syndrome, dry eye syndrome accompanied by allergic conjunctivitis, tear reduction in visual display terminal (VDT) workers, dry environment, and wearing of contact lenses. The dry eye syndrome may include a disorder of the tear film due to tear deficiency or excessive evaporation, and may also include symptoms of ocular discomfort which causes damage to the interpalpebral ocular surface. Further, the dry eye syndrome is a multifactorial disease of tears and ocular surface that results in symptoms of discomfort, visual disturbance, and tear film instability with potential damage to the ocular surface. It is accompanied by increased osmolarity of the tear film and inflammation of the ocular surface. Further, the dry eye syndrome involves aqueous tear-deficient dry eye syndrome and evaporative dry eye syndrome. The aqueous tear-deficient dry eye syndrome may include diseases caused by a failure of lacrimal tear secretion. Further, the evaporative dry eye syndrome may include diseases caused by excessive water loss from the exposed ocular surface in the presence of normal lacrimal secretory function.

In a specific embodiment, the composition may increase tear secretion or may restore corneal injury. The corneal injury means that the thickness of the corneal surface becomes thinner, as compared with a normal control, due to excessive tear drying, which may be improved by the composition of the present disclosure.

The composition may be used after being formulated into various preparations according to a common method. For example, the composition may be an oral preparation. The oral preparation may be one or more selected from the group consisting of a tablet, granules, a pill, a capsule, a liquid formulation, and a jelly.

The composition may further include a pharmaceutically acceptable carrier, excipient, or diluent. The pharmaceutically acceptable carrier, excipient, or diluent may include any one or more selected from the group consisting of lactose, dextrose, sucrose, calcium silicate, cellulose, methylcellulose, microcrystalline cellulose, polyvinylpyrrolidone, water, methylhydroxy benzoate, propylhydroxy benzoate, talc, magnesium stearate, and mineral oil, but is not limited thereto.

Those skilled in the art may readily determine and prescribe an administration dose which is effective for the treatment. Administration of the pharmaceutical composition according to the present disclosure may be performed once daily or in a few divided doses. Therefore, the above administration dose does not limit the scope of the present disclosure in any aspect. The administration dose of the pharmaceutical composition may be 1 mg/kg/day to 1,000 mg/kg/day.

As used herein, the term "active ingredient" or "effective amount" may mean any amount of the composition sufficient to alleviate, inhibit, or prevent a disease, a disorder, or a disease condition, or one or more symptoms thereof.

As used herein, the term "preventing" refers to all actions of approaching a normal control by restraining or retarding symptoms of the eye disease by administering the composition according to the present disclosure.

As used herein, the term "treating" means all actions by which symptoms of the eye disease become equal to those of a normal control, have taken a turn for the better or been modified favorably by administering the composition according to the present disclosure.

As used herein, the term "pharmaceutical composition" refers to those prepared for preventing or treating a disease.

Another aspect provides a method of treating an eye disease in an individual, the method including administering, to the individual, the pharmaceutical composition in an effective amount for preventing or treating the eye disease.

As used herein, the term "administering" may be used interchangeably with "introducing", and "transplanting", and refers to placement of the composition according to a specific embodiment into an individual by a method or route which results in at least partial localization of the composition according to a specific embodiment at a desired site. An extract of the composition according to a specific embodiment or at least portion of the extract component may be administered via any appropriate route which results in delivery to a desired site in the individual that survives.

Administration may be performed by a method known in the art. For example, the composition may be directly administered to an individual by any means via a route such as intravenous, intramuscular, oral, transdermal, mucosal, intranasal, intratracheal, or subcutaneous administration. The administration may be performed systemically or locally.

The individual may be a mammal. The mammal may be a human, a dog, a cat, a cow, a goat, or a pig.

The administration may be performed by any general route as long as it may reach a target tissue. For example, it may be administered via a route such as eye drop administration, intraperitoneal administration, intravenous administration, intramuscular administration, subcutaneous administration, intradermal administration, transdermal patch administration, oral administration, etc., and specifically, via the route of eye drop administration, as needed.

It is understood that those mentioned for the description of the composition, among the terms or elements mentioned in the treatment method, are the same as in those mentioned in the above description of the composition.

Described is a health functional food composition for preventing or improving the eye disease or for improving eye function, the health functional food composition including *Tisochrysis lutea* as an active ingredient.

The algae, active ingredient, eye disease, and preventing are as described above.

As used herein, the phrase "improving the eye disease" may mean alleviating symptoms of the eye disease that have already occurred.

As used herein, the phrase "improving eye function" may mean reducing or alleviating the degree of negative symptoms, such as eye discomfort when recognizing the presence or shape of an object.

The health functional food composition includes drinks, meats, sausages, breads, biscuits, rice cakes, chocolates, candies, snacks, confectionery, pizzas, ramen noodles, other noodles, gums, dairy products such as ice-creams, various soups, beverages, alcoholic beverages, and vitamin complexes. The health functional food includes all commonly accepted health functional foods.

The active ingredient of the present disclosure may be added to a food as it is or may be used with other foods or food ingredients, and may be appropriately used according to a common method. A mixing amount of the active ingredient may be appropriately determined according to its purpose of use (for prevention or improvement). In general, the active ingredient in the health functional food may be added in an amount of 0.1 part by weight to 90 parts by weight, based on the total weight of the food. However, in the case of long-term intake for health and hygiene or for controlling health conditions, the amount may be less than the above range. The active ingredient may be used in an amount exceeding the above range.

The health functional beverage composition of the present disclosure has no particular limitation on other ingredients, except that the active ingredient is included at a predetermined ratio as an essential ingredient. It may include various flavoring agents, natural carbohydrates, etc. as additional ingredients, as in common beverages. Examples of the above-described natural carbohydrates include common sugars such as monosaccharides, for example, glucose, fructose, etc.; disaccharides, for example, maltose, sucrose, etc.; and polysaccharides, for example, dextrin, cyclodextrin, etc., and sugar alcohols such as xylitol, sorbitol, erythritol, etc. In addition to those described above, natural flavoring agents (thaumatin, stevia extract (e.g., rebaudioside A, glycyrrhizin, etc.)) and synthetic flavoring agents (e.g., saccharin, aspartame, etc.) may be used as the flavoring agents. A proportion of the natural carbohydrate may be about 1 g to 20 g, or about 5 g to 12 g per 100 ml of the composition of the present disclosure.

In addition to those described above, the active ingredient of the present disclosure may include various nutrients, vitamins, minerals (electrolytes), flavors such as synthetic flavors, natural flavors, etc., coloring agents, enhancers (cheese, chocolate, etc.), pectic acid and salts thereof, alginic acid and salts thereof, organic acids, protective colloidal thickeners, pH regulators, stabilizers, antiseptics, glycerin, alcohols, and carbonating agents used in carbonated drinks, etc. In addition, the active ingredient of the present disclosure may include natural fruit juice and fruit flesh for preparation of fruit beverages and vegetable beverages. These ingredients may be used alone or in combination thereof. A proportion of these additives is, for example, selected from the range of about 0.1 parts by weight to about 20 parts by weight, based on 100 parts by weight of the active ingredient of the present disclosure.

### BRIEF DESCRIPTION OF THE DRAWINGS

The above and other aspects, features, and advantages of certain embodiments of the disclosure will be more apparent from the following description taken in conjunction with the accompanying drawings, in which:

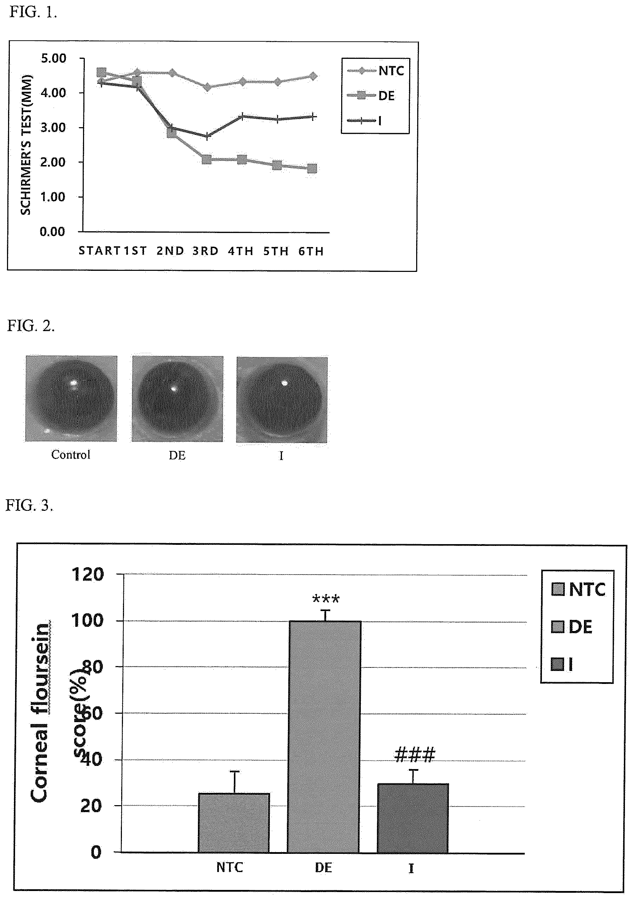

FIG. 1 is a graph showing results of measuring tear production of mice over time (week) by using a Schirmer test method (NTC represents a non-treated normal control group, DE represents a disease group with scopolamine-induced dry eye syndrome, and I represents a *Tisochrysis lutea*-treated group with scopolamine-induced dry eye syndrome);
FIG. 2 shows photographs showing the degree of corneal injury in mice, determined by using a fluorescence staining method;
FIG. 3 is a graph showing results of determining corneal injury in mice by using a fluorescence staining method (NTC represents a non-treated normal control group, DE represents a disease group with scopolamine-induced dry eye syndrome, and I represents a *Tisochrysis lutea*-treated group with scopolamine-induced dry eye syndrome, and *** represents p<0.001, and ### represents P<0.001);

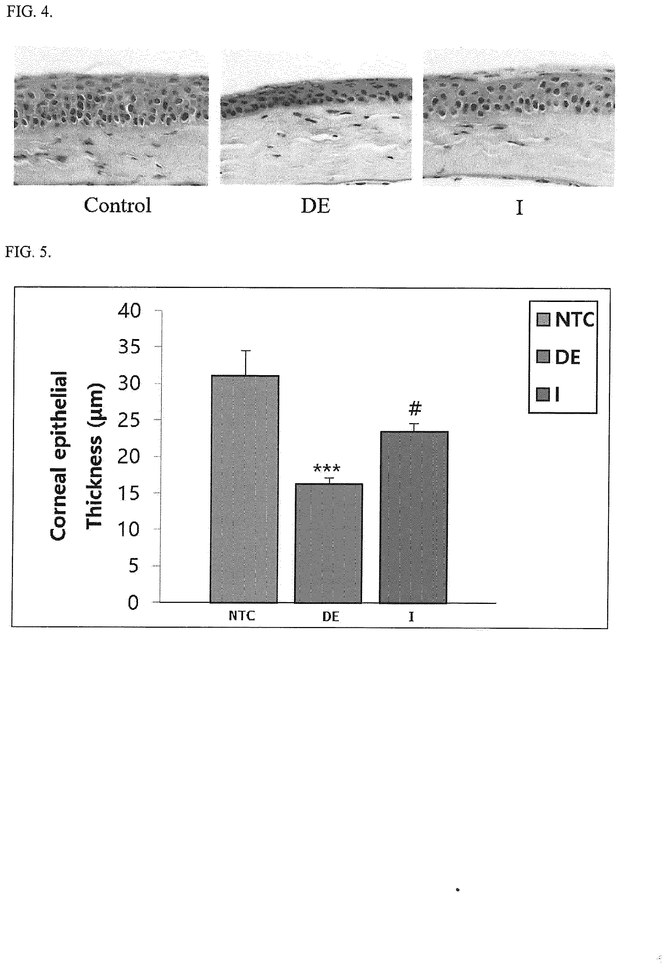

FIG. 4 shows photographs which show the corneal thicknesses of mice, determined by an H&E staining method; and
FIG. 5 is a graph showing results of measuring the corneal thicknesses of mice (NTC represents a non-treated normal control group, DE represents a disease group with scopolamine-induced dry eye syndrome, and I represents a *Tisochrysis lutea*-treated group with scopolamine-induced dry eye syndrome, and # represents P<0.05, and *** represents p<0.001).

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

### Example 1: Therapeutic Effect of Tisochrysis lutea on Eye Disease

### 1.1 Tisochrysis lutea

*Tisochrysis lutea* included in a composition of the present disclosure was purchased from Komipharm.

### 1.2 Preparation of animal model with scopolamine-induced dry eye syndrome

7-week-old female BALB/c mice were purchased and acclimated to an air-controlled animal facility with a temperature of 24±2°C, humidity of 50±10%, and 12 hr light/12 hr dark cycle. Food and water were provided ad libitum. After a week of domestication, 200 µl of 2.5 mg/mL scopolamine was subcutaneously injected twice a day for 2 weeks.

### 1.2.1 Design of animal test

To examine an effect of an algae extract on scopolamine-induced dry eye syndrome, experimental animals of Section 2.1 were used. Five mouse individuals randomly assigned to each experimental group were constructed as follows: a non-treated normal control group (NTC), a disease group with scopolamine-induced dry eye syndrome by a method of Section 2.1 (DE), and a *Tisochrysis lutea*-administered group with scopolamine-induced dry eye syndrome (I), i.e., one normal control group, one disease group, and three administered groups. *Tisochrysis lutea* was orally administered to each mouse once a day at a concentration of 300 mg/kg.

### 1.2.2 Measurement of tear production and Measurement of corneal injury

Effects of the algae extract on tear production and corneal injury in scopolamine-induced dry eye syndrome were examined. At 0 week, 1 week, 2 weeks, 3 weeks, 4 weeks, 5 weeks, and 6 weeks after drug administration, the design of animal test of Section 1.2.1 was used to measure tear production by a Schirmer test. Before sacrificing the mice, corneal injury in the eyes of the mice was examined by a fluorescence staining method.

In detail, tear production was measured for five mice each group seven times by the Schirmer test: a sterile standardized phenol red thread (ZONE-QUICK, Japan) was placed in the lateral canthus of each mouse for 15 seconds, and then the wet portion was measured. FIG. 1 is a graph showing results of measuring tear production of mice over time (week) by using the Schirmer test method (NTC represents the non-treated normal control group, DE represents the disease group with scopolamine-induced dry eye syndrome, and I represents the *Tisochrysis lutea*-treated group with scopolamine-induced dry eye syndrome).

The degree of corneal injury of the mice was examined by performing the fluorescence staining for the eyes of five mice each group. One drop (about 15 µl to about 20 µl) of fluorescein was applied to the corneal surface of each mouse to coat the tear film, followed by visualizing the cornea with blue light. When the injured corneal surface was stained with fluorescein, it appears green under blue light. The corneal surface was photographed, and a percentage of the green-stained injured cornea area to the entire area of the cornea was calculated using Image J to determine the degree of injury. FIG. 2 shows photographs showing the degree of corneal injury in mice, determined by using a fluorescence staining method. FIG. 3 is a graph showing results of determining corneal injury in mice by using a fluorescence staining method (NTC represents a non-treated normal control group, DE represents a disease group with scopolamine-induced dry eye syndrome, and I represents a *Tisochrysis lutea*-treated group with scopolamine-induced dry eye syndrome, and *** represents p<0.001, and ### represents P<0.001).

As a result, the *Tisochrysis lutea*-treated group (I) showed about 60% increase in the tear production, as compared with the DE group (FIG. 1), and showed repair of corneal injury in a similar level to the normal control group, as compared with the DE group (FIGS. 2 and 3).

These results indicate that *Tisochrysis lutea* increased tear production and prevented corneal injury, thereby exhibiting prophylactic or therapeutic effects on dry eye syndrome.

### 1.2.3 Measurement of corneal thickness

Effects of *Tisochrysis lutea* on changes in corneal thickness in scopolamine-induced dry eye syndrome were examined. In detail, the design of animal test of Section 1.2.1 was used to stain the eye ball and tear gland tissues by an H&E staining method.

In detail, to measure changes in the corneal epithelial tissues and tear glands, one eye and tear gland of the sacrificed mouse were prepared in paraffin blocks, and then paraffin blocks were cut to 3 µm, and paraffin was removed for 3 minutes three times using a xylene solution. Ethanol, acetic acid, and glycerol were added to a 10 (v/v)% ammonium solution to prepare a hematoxylin solution, which was used to stain for 10 minutes the tissue slides from which paraffin had been removed. The stained slides were washed for 1 minute with distilled water, and then immersed in 1 (v/v)% HCl-70 (v/v)% ethanol for 5 seconds, and then immersed in distilled water for 5 minutes. Then, the slides were treated with a 0.3% ammonia solution for 2 minutes and washed with distilled water. The washed tissues were subjected to eosin staining for 2 minutes using an eosin staining solution which was prepared by using 1 (v/v)% eosin, ethanol, and acetic acid, and then washed once with 50%, 70%, 90%, 95%, 100% ethanol, xylene solution (2 minutes/time), respectively. After Canadian balsam was dropped on each washed slide, and the slide was covered with a cover slide to harden it, changes in the cornea and tear gland were measured using an inverted microscope (TE-2000U, Nikon, Tokyo, Japan). Five randomly selected regions of the cornea were photographed, and the thickness of the epithelial layer was measured as a mean value using Image J program. FIG. 4 shows photographs which show the corneal thicknesses of mice, determined by an H&E staining method. FIG. 5 is a graph showing results of measuring corneal thickness of mice (NTC represents a non-treated normal control group, DE represents a disease group with scopolamine-induced dry eye syndrome, and I represents a *Tisochrysis* lutea-treated group with scopolamine-induced dry eye syndrome, and # represents P<0.05, and *** represents p<0.001).

As a result, it was confirmed that the corneal thickness in the *Tisochrysis lutea*-treated group (I) was increased, as compared with the DE group (FIGS. 4 and 5).

These results indicate that *Tisochrysis lutea* repaired the corneal thickness, thereby exhibiting prophylactic or therapeutic effects on dry eye syndrome.

According to a pharmaceutical composition and a health functional food composition according to an aspect, tear production may be increased or corneal injury may be repaired, and thus the compositions may be usefully applied to preventing, treating, or improving eye diseases.

It should be understood that embodiments described herein should be considered in a descriptive sense only and not for purposes of limitation. Descriptions of features or aspects within each embodiment should typically be considered as available for other similar features or aspects in other embodiments.

## Claims

1. A composition comprising *Tisochrysis lutea* for use in preventing or treating dry eye syndrome.

2. The composition for use according to claim 1, wherein the *Tisochrysis lutea* is comprised in an amount of 0.001% by weight to 90.0% by weight with respect to the total weight of the composition.

3. The composition for use according to claim 1 to 2, wherein the dry eye syndrome is caused by one or more selected from the group consisting of alacrima, corneal xerosis, Sjogren's syndrome, keratoconjunctivitis sicca, Stevens-Johnson syndrome, ocular pemphigoid, dry eye syndrome after ophthalmic surgery, dry eye syndrome accompanied by allergic conjunctivitis, tear reduction in visual display terminal (VDT) workers, dry environment, and wearing of contact lenses.

4. The composition for use according to any one of claims 1 to 3, wherein the composition increases tear production or repairs corneal injury.

5. The composition for use according to any one of claims 1 to 4, wherein the composition is in the form of an oral or eye drop formulation.

## Patentansprüche

1. Zusammensetzung, umfassend *Tisochrysis lutea* zur Verwendung bei der Prävention oder Behandlung des Syndroms des trockenen Auges.

2. Zusammensetzung zur Verwendung nach Anspruch 1, wobei *Tisochrysis lutea* in einer Menge von 0,001 Gew.-% bis 90,0 Gew.-% in Bezug auf das Gesamtgewicht der Zusammensetzung umfasst ist.

3. Zusammensetzung zur Verwendung nach Anspruch 1 oder 2, wobei das Syndrom des trockenen Auges durch mindestens eine Ursache ausgelöst ist, die aus der Gruppe ausgewählt ist, die aus Alakrimie, kornealer Xerose, Sjögren-Syndrom, Keratokonjunktivitis sicca, Stevens-Johnson-Syndrom, okulärem Pemphigoid, Syndrom des trockenen Auges nach einer Augenoperation, Syndrom des trockenen Auges begleitet von allergischer Konjunktivitis, Tränenreduktion bei Personen, die mit Bildschirmgeräten (BSG) arbeiten, trockener Umgebung und Tragen von Kontaktlinsen besteht.

4. Zusammensetzung zur Verwendung nach einem der Ansprüche 1 bis 3, wobei die Zusammensetzung die Tränenproduktion steigert oder Hornhautverletzungen repariert.

5. Zusammensetzung zur Verwendung nach einem der Ansprüche 1 bis 4, wobei die Zusammensetzung in der Form einer oralen Formulierung oder Augentropfen-Formulierung vorliegt.

## Revendications

1. Composition, comprenant de la *Tisochrysis lutea* pour l'utilisation dans la prévention ou le traitement du syndrome de l'oeil sec.

2. Composition pour l'utilisation selon la revendication 1, dans laquelle la *Tisochrysis lutea* est comprise dans une quantité de 0,001 % en poids à 90,0 % en poids par rapport au poids total de la composition.

3. Composition pour l'utilisation selon la revendication 1 ou 2, dans laquelle le syndrome de l'oeil sec est provoqué par un ou plusieurs chosi(s) dans le groupe qui se compose de l'alacrymie, du xérosis cornéen, du syndrome de Sjögren, de la kératoconjonctivite sicca, du syndrome de Stevens-Johnson, de la pemphigoïde oculaire, du syndrome de l'oeil sec après une opération ophtalmique, du syndrome de l'oeil sec accompagné d'une conjonctivite allergique, de la réduction des larmes chez des personnes travaillant avec des terminaux à écran de visualisation (TEV), d'un environnement sec et du port de lentilles de contact.

4. Composition pour l'utilisation selon l'une quelconque des revendications 1 à 3, dans laquelle la composition augmente la production de larmes ou répare des lésions de la cornée.

5. Composition pour l'utilisation selon l'une quelconque des revendications 1 à 4, dans laquelle la composition est sous la forme d'une formulation orale ou de collyres.
